# EUROPEAN PATENT SPECIFICATION

(11) **EP 0 416 466 B1**
(45) Date of publication and mention of the grant of the patent: **08.03.1995**
(21) Application number: 90116658.7
(22) Date of filing: 30.08.1990
(51) Int. Cl.: G01N 33/543, G01N 33/569

(54) **Nonspecific adsorption-free method for assaying antibody and assay reagent**
Nichtspezifische adsorptionsfreie Methode zur Bestimmung eines Antikörpers und Bestimmungsreagentien
Méthode pour le dosage d'anticorps débarrassée d'adsorption non-spécifique et réactif de dosage

(30) Priority: 05.09.1989 JP 229753/89
(43) Date of publication of application: 13.03.1991
(73) Proprietor: Eisai Co., Ltd., Tokyo (JP)
(72) Inventor: Sawada, Takashi, Tsukuba-shi, Ibaraki (JP); Obara, Takashi, Tsukuba-shi, Ibaraki (JP); Miyoshi, Isao, Koichi-shi, Koichi (JP); Taguchi, Hirokuni, Koichi-shi, Koichi (JP)
(74) Representative: Kolb, Helga, Dr. Dipl.-Chem.

(56) References cited:
- EP-A- 0 234 941
- WO-A-90/09594
- JP-A- 8 954 356

## Description

This invention relates to a nonspecific adsorption-free method for assaying an antibody and an assay reagent. More particularly, it relates to an assay method comprising contacting a solid phase with a serum specimen to thereby assay an antibody in the specimen and an assay reagent, wherein nonspecific adsorption caused by said contact is eliminated so as to elevate the assay sensitivity.

It is frequently observed in an immunological assay with the use of a solid phase that nonspecific adsorption of an antibody on the solid phase leads to errors in the assay results (refer to, for example, the following references):
1) E. J. Shillitoe: J. of Virological Methods, 4, 241 - 248 (1982);
2) T. Sawada et al.: Rinsho to Kenkyu, 62 (1), 311 - 318 (1985);
3) T. Sawada et al.: Rinsho to Kenkyu, 63 (11), 3818 - 3822 (1986); and
4) Japanese Patent Laid-Open No. 54356/1989.

Namely, these references describe that denatured or aggregated IgG (which will be referred to as the denatured antibody hereinbelow) formed in a serum specimen stored for a long time [reference 1)] or thermally immobilized serum specimens [references 2) and 3)] would be nonspecifically adsorbed on a solid phase and thus lead to errors in the assay results. These references further describe that these errors in the assay results caused by the nonspecific adsorption of the denatured antibody can be eliminated by conducting a pretreatment (addition of kaolin) [reference 1)] or by using a cup washer of excellent performance or appropriately setting a cut-off value [references 2) and 3)]. According to the reference 4), on the other hand, a labeled antibody added for the assay would be nonspecifically adsorbed on a solid phase and thus lead to errors in the assay results. The reference 4) discloses that these errors caused by the nonspecific adsorption of the labeled antibody can be suppressed by reacting the solid phase with the labeled antibody in a buffer solution of a pH value of from 8.0 to 9.5.

The nonspecific adsorptions of the denatured antibody and labeled antibody described in the above references 1) to 4) are seemingly the same with each other in terms of the adsorption of an antibody on a solid phase. However they should be treated as completely different phenomenons in practical assay procedures. This is not only because the denatured antibody is different from a labeled antibody in its nature, but also because the former nonspecific adsorption occurs in a reaction (a so-called first reaction) when a serum specimen is added to a solid phase in an assay according to the solid phase method, while the latter nonspecific adsorption occurs in another reaction (a so-called second reaction) when a labeled antibody is added following the completion of the first reaction. Therefore the treatment for the elimination of the former nonspecific adsorption completely differs from the one for the suppression of the latter nonspecific adsorption [for example, the one described in the reference 4)] and cannot be easily conceived therefrom. Although the references 1) to 3) provide treatments for the elimination of the nonspecific adsorption of the denatured antibody, it has been required to establish a more simple and accurate process for that. The present invention has been completed in order to solve this problem.

The present inventors have conducted extensive studies in order to solve the above problem. As a result, they have found out that the nonspecific adsorption of the denatured antibody on a solid phase, observed when a serum specimen is added to the solid phase, can be eliminated by conducting said addition at a pH value of from 9 to 10, thus completing the present invention.

Accordingly, the present invention provides an immunoassay method to determine an antibody in a serum specimen comprising adding the serum specimen to an antigen coated solid phase to thereby assay said antibody, characterized in that said addition is conducted at a pH value of from 9 to 10. There is further provided an assay reagent for assaying an antibody in a serum specimen by adding said specimen to a solid phase, wherein a buffer solution of a pH value of from 9 to 10 is essentially required for the addition.

The assay method according to the invention comprises the steps of adding a serum specimen to a solid phase at a pH value of 9 to 10 and assaying an antibody in the specimen. The antibody may be adult T cell leukemia virus antibody (HTLV-I antibody).

The invention also provides an assay reagent kit for an immunoassay method to determine an antibody in a serum specimen comprising adding the serum specimen to an antigen coated solid phase, which comprises microtiter wells as solid phase, a buffer solution to adjust the pH at 9 to 10, an enzyme labeled second antibody and a substrate solution.

The assay reagent kit for assaying an antibody in a serum specimen by adding the specimen to a solid phase comprises microtiter wells, a buffer solution to adjust the pH at 9 to 10, an enzyme-labeled second anti-body and a substrate solution.

### Brief Description of the Drawings:

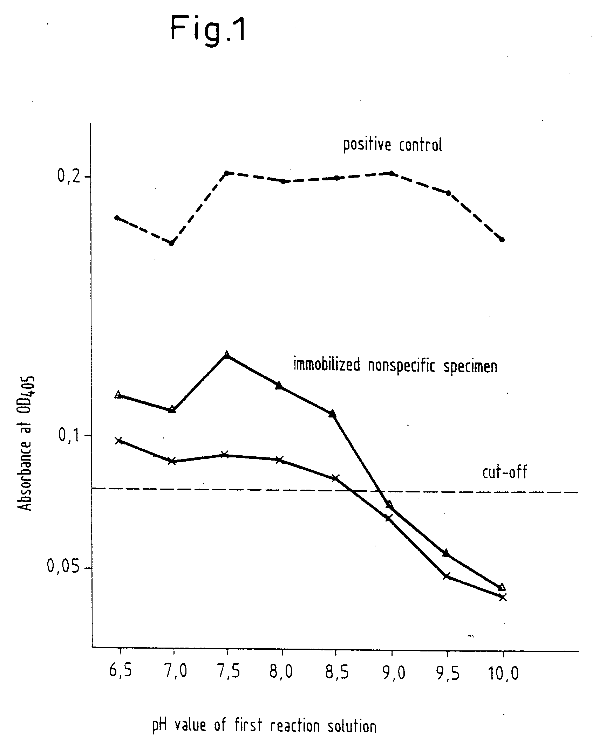

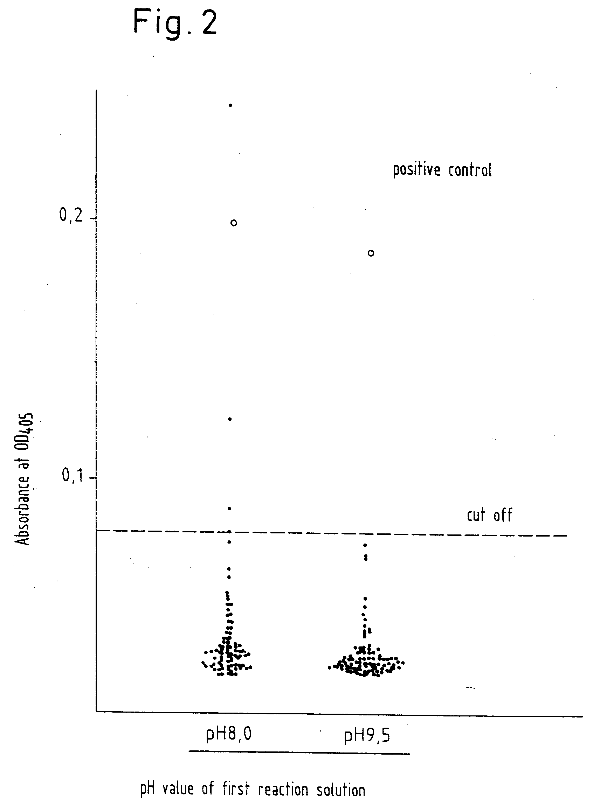

Fig. 1 is a graph showing the results of Test Example 1, while Fig. 2 is a graph showing the results of Test Example 2.

Now the present invention will be described in detail.

The antibody to be assayed in the present invention is not particularly restricted. That is to say, the antibody to be assayed in the present invention may be an arbitrary one, since the present invention relates to the nonspecific adsorption of the denatured antibody contained in a serum specimen, in particular, a human serum specimen and provides a means for solving this problem.

The specimen is a serum specimen. Thus either serum or blood may be used as a sample.

As the solid phase, a microtiter plate cup or glass beads for immunoassay may be used. The surface of the solid phase has been preliminarily coated with an antigen in a conventional manner.

The assay method and assay reagent of the present invention are utilized in immunoassay methods wherein a solid phase is used, for example, enzyme immunoassay, radioimmunoassay and passive agglutination. Common procedures of these methods, which are widely known per se, may be applied to the present invention. In the case of enzyme immunoassay, for example, the assay method of the present invention may be conducted as follows.

The components to be used in the assay include a solid phase, an antigen, a serum specimen, an enzyme-labeled antibody, a substrate, a buffer solution, a diluent, a ceasing solution and a standard antibody. First, the solid phase is coated with an antigen and the serum specimen, which is optionally diluted with normal rabbit serum, is added thereto. After thoroughly washing, the enzyme-labeled antibody is added. After thoroughly washing, the substrate is added and the reaction is ceased. Next, the amount of the decomposed substrate is determined. In this case, the present invention is characterized in that said serum specimen is added at a pH value of from 9 to 10. In order to achieve said condition an alkali or an acid is added for the adjustment of the PH value. A buffer solution is usually employed for that.

The assay reagent of the present invention is prepared exclusively for the embodiment of the assay method of the present invention. In addition to the buffer solution, which is an essential component, the assay reagent of the present invention may comprise a combination of several components which are arbitrarily selected. Any buffer solution may be used as long as it can maintain a pH value of from 9 to 10. For example, a phosphate buffer solution, a tris hydrochloride buffer solution or a borate buffer solution may be used. The concentration usually ranges of from 0.05 to 0.1 M, though it may be determined depending on each case. It is also possible to provide the buffer solution in the form of a solid component thereof which is simultaneously dissolved in water to thereby give the aimed buffer solution. It substantially falls within the scope of the present invention.

As will be shown in Example and Test Examples hereinafter, the application of the present invention to the assay of adult T cell Leukemia virus antibody (HTLV-L antibody) can give excellent results.

According to the present invention, nonspecific adsorption of the denatured antibody in a serum specimen, in particular, those which have been stored for a long time or thermally immobilized can be eliminated. Accordingly, the function of the present invention resides in the increase in the detection sensitivity through the elimination of nonspecific adsorption.

To further illustrate the present invention, and not by way of limitation, the following Example will be given.

### Example 1

HTLV-1 antigen was dissolved in a 0.05 M tris hydrochloride buffer solution (pH 8.0) to give a concentration of 1 »g/ml. 100-»l portions of the solution thus obtained were pipetted into microtiter wells and allowed to stand at 4°C overnight. After washing the wells with distilled water, 100-»l portions of normal rabbit serum, which had been adjusted to a pH of 9.5 with a 0.1 M borate buffer solution, were added as a solution for the first reaction. Next, 20-»l portions of a serum specimen were added thereto and incubated at 37°C for 60 minutes (the first reaction). The inside of each well was washed with physiological saline containing 0.01% of Tween® 20. Then 100-»l portions of an alkaline phosphatase-labeled antihuman globulin antibody were added to the wells and incubated at 37°C for 60 minutes (the second reaction). After washing the inside of each well with physiological saline containing 0.01% of Tween 20, 100-»l portions of a substrate solution (4 mg/ml of p-nitrophenyl phosphate) were added to the wells and incubated at 37°C for 30 minutes (the third reaction). 100-»l portions of a 1N NaOH solution were added to the wells to thereby cease the reaction. Then the absorbance of each reaction mixture was measured at 405 nm. The cut-off value was 0.08 (the absorbance at 405 nm). Namely, reaction mixtures showing an absorbance exceeding this value were judged to be positive for the HTLV-I antibody, while those showing absorbances lower that it were judged to be negative therefor.

To further illustrate the effects of the present invention, the following Test Examples will be given.

### Test Example 1

a) Sample
   The serums of two normal subjects negative for the HTLV-I antibody were thermally immobilized by heating at 56°C for 30 minutes. The specimens showing a nonspecific reaction thus obtained (immobilized nonspecific specimens 1 and 2) and the serum of an HTLV-I carrier (an HTLC-1 antibody positive control) were employed.
b) Method
   Each sample described in the above item a) was treated by the method described in Example 1 to thereby examine the immunological reactivity to HTLV-1. In this test the following solutions (a) to (c), whose pH values had been adjusted, were employed as the first reaction solution described in Example 1;
   (a) first reaction solutions buffered with a 0.1 M phosphate buffer solution (normal rabbit serum buffered to pH 6.5, 7.0 and 7.5),
   (b) first reaction solutions buffered with a 0.05 M tris hydrochloride buffer solution (normal rabbit serum buffered to pH 8.0 and 8.5).
   (c) first reaction solutions buffered with a 0.1 M borate buffer solution (normal rabbit serum buffered to pH 9.0, 9.5 and 10.0).
c) Result
   Fig. 1 gives the results.

As the figure obviously shows, the immobilized nonspecific specimens were judged to be positive (pseudopositive), since their absorbances exceeded the cut-off value when the pH value of the first reaction solution was 6.5 to 8.5. When the pH value of the first reaction solution increased to 9.0 to 10.0, however, the absorbances of these specimens were lower than the cut-off value and thus they were judged to be negative. On the other hand, the absorbance of the positive control remained higher than the cut-off value at any pH value of the first reaction solution. Thus it never turned negative. These results indicate that the nonspecific reaction would be effectively inhibited by increasing the pH value of the first reaction solution to 9.0 to 10.0.

In this test, a first reaction solution buffered with a borate buffer solution (pH 9.0 - 10.0) was employed. In addition, the use of those buffered with a glycine/sodium hydroxide buffer solutions a veronal hydrochloride buffer solution and a carbonate/bicarbonate buffer solution also gave similar results.

### Test Example 2

a) Sample
   90 normal human serum specimens negative for the HTLV-1 antibody and the serum of an HTLV-1 carrier (as an HTLV-1 antibody positive control) were employed.
b) Method
   Each sample described in the above item a) was treated by the method described in Example 1 to thereby examine the immunological reactivity to HTLV-1. In this test the first reaction solutions of pH 8.0 and pH 9.5 described in Test Example 1 were employed as the first reaction solution.
c) Result
   Fig. 2 gives the results.

As this figure obviously shows, four specimens (4.4%) showed absorbances exceeding the cut-off value and thus judged to be positive (pseudopositive), when the first reaction solution of pH 9.0 was employed. When the first reaction solution of pH 9.5 was employed, however, all specimens showed absorbances lower than the cut-off value. In this case, none was judged to be pseudopositive based on a nonspecific reaction. The absorbance of the positive control observed with the use of the first reaction solution of pH 9.5 was somewhat lower than that observed with the use of the first reaction solution of pH 8.0, though the difference was small.

These results indicate that the nonspecific reaction would be effectively inhibited by increasing the pH value of the first reaction solution to 9.5, when normal human specimens were employed.

## Claims

1. An immunoassay method to determine an antibody in a serum specimen comprising adding the serum specimen to an antigen coated solid phase to thereby assay said antibody, characterized in that
said addition is conducted at a pH value of from 9 to 10.

2. The method as claimed in claim 1, in which the antibody is adult T cell leukemia virus antibody (HTLV-I antibody).

3. An assay reagent kit for an immunoassay method to determine an antibody in a serum specimen comprising adding the serum specimen to an antigen coated solid phase, which comprises microtiter wells as solid phase, a buffer solution to adjust the pH at 9 to 10, an enzyme labeled second antibody and a substrate solution.

## Patentansprüche

1. Immunoassay-Verfahren zur Bestimmung eines Antikörpers in einer Serumprobe, bei dem die Serumprobe zur Bestimmung des Antikörpers zu einer mit Antigen überzogenen festen Phase gegeben wird,
dadurch **gekennzeichnet**, daß die Zugabe bei einem pH-Wert von 9 bis 10 erfolgt.

2. Verfahren nach Anspruch 1, bei dem der Antikörper Erwachsenen-T-Zellen-Leukämievirus-Antikörper (HTLV-I-Antikörper) ist.

3. Analysereagenziensatz für ein Immunoassay-Verfahren zur Bestimmung eines Antikörpers in einer Serumprobe, bei dem die Serumprobe zu einer mit Antigen überzogenen festen Phase gegeben wird, welcher Mikrotiter-Behälter (microtiter wells) als feste Phase, eine Pufferlösung zur Einstellung des pH auf 9 bis 10, einen zweiten, mit Enzym markierten Antikörper und eine Substratlösung umfaßt.

## Revendications

1. Procédé d'immunodosage pour déterminer un anticorps dans un échantillon de sérum comportant l'addition de l'échantillon de sérum à une phase solide recouverte d'un antigène pour ainsi doser cet anticorps, caractérisé en ce que ladite addition est effectuée à un pH d'une valeur comprise entre 9 et 10.

2. Procédé selon la revendication 1, dans lequel l'anticorps est l'anticorps du virus de la leucémie à lymphocytes T adulte (anticorps HTLV-I).

3. Trousse de réactif de dosage pour un procédé d'immunodosage pour déterminer un anticorps dans un échantillon de sérum comportant l'addition de l'échantillon de sérum à une phase solide recouverte d'un antigène, qui comprend des puits de microtitration comme phase solide, une solution tampon pour ajuster le pH à une valeur comprise entre 9 et 10, un second anticorps marqué à l'aide d'une enzyme et une solution de substrat.
